# EUROPÄISCHE PATENTANMELDUNG

(11) **EP 4 520 252 A2**
(43) Veröffentlichungstag der Anmeldung: **12.03.2025**
(21) Anmeldenummer: 24198836.9
(22) Anmeldetag: 06.09.2024
(51) Int. Cl.: A61B 1/05, A61B 1/06, A61B 1/12, A61B 1/045

(54) **MEDIZINISCHE BILDGEBUNGSVORRICHTUNG, INSBESONDERE ENDOSKOP ODER EXOSKOP, SOWIE VERFAHREN ZUM STEUERN UND/ODER REGELN EINER MEDIZINISCHEN BILDGEBUNGSVORRICHTUNG**

(30) Priorität: 07.09.2023 DE 102023124088
(71) Anmelder: Karl Storz SE & Co. KG, 78532 Tuttlingen (DE)
(72) Erfinder: Forster, Jonas, 78532 Tuttlingen (DE); Hedemann, Lars, 78532 Tuttlingen (DE)
(74) Vertreter: Eichelhardt, Frank Robert

(57) **Zusammenfassung**

Die Erfindung betrifft eine medizinische Bildgebungsvorrichtung, insbesondere Endoskop oder Exoskop, mit einem Schaft, einer Lichtquelle zum Beleuchten eines Betrachtungsbereiches und einem Bildsensor zum Aufnehmen des Betrachtungsbereiches, wobei der Schaft einen distalen Abschnitt, einen proximalen Abschnitt, eine vom distalen Abschnitt zum proximalen Abschnitt verlaufende Längsachse, eine am distalen Abschnitt angeordnete Stirnfläche und eine entlang der Längsachse verlaufende, einer Umgebung zugewandte Schaftwandung aufweist, die Lichtquelle und der Bildsensor in einem distalen Abschnitt des Schaftes aufgenommen sind und eine durch die Lichtquelle und/oder durch den Bildsensor abgegebene Betriebswärme mittels einer Wärmesenke in Richtung eines proximalen Bereiches des Schaftes abgeführt wird und der Lichtquelle und dem Bildsensor ein Wärmeübertragungselement zum Leiten der Betriebswärme von der Lichtquelle und/oder vom Bildsensor an die mit dem Wärmeübertragungselement wärmeleitend verbundene Wärmesenke zugeordnet ist. Des Weiteren betrifft die Erfindung ein Verfahren zum Steuern und/oder Regeln einer medizinischen Bildgebungsvorrichtung.

## Beschreibung

Die Erfindung betrifft eine medizinische Bildgebungsvorrichtung, insbesondere ein Endoskop oder ein Exoskop, mit einem Schaft, einer Lichtquelle zum Beleuchten eines Betrachtungsbereiches und einem Bildsensor zum Aufnehmen des Betrachtungsbereiches, wobei der Schaft einen distalen Abschnitt, einen proximalen Abschnitt, eine vom distalen Abschnitt zum proximalen Abschnitt verlaufende Längsachse, eine am distalen Abschnitt angeordnete Stirnfläche und eine entlang der Längsachse verlaufende, einer Umgebung zugewandte Schaftwandung aufweist, die Lichtquelle und der Bildsensor an einem distalen Abschnitt des Grundkörpers aufgenommen sind und eine durch die Lichtquelle und/oder durch den Bildsensor abgegebene Betriebswärme mittels einer Wärmesenke in Richtung eines proximalen Bereichs des Grundkörpers abgeführt wird und der Lichtquelle und dem Bildsensor ein Wärmeübertragungselement zum Leiten der Betriebswärme von der Lichtquelle und/oder vom Bildsensor an die mit dem Wärmeübertragungselement wärmeleitend verbundene Wärmesenke zugeordnet ist. Weiterhin betrifft die Erfindung ein Verfahren zum Steuern und/oder zum Regeln einer solchen medizinischen Bildgebungsvorrichtung.

Bekannte medizinische Bildgebungsvorrichtungen, insbesondere Endoskope oder Exoskope, weisen häufig eine als "Heatpipe" ausgeführte Wärmesenke innerhalb des Schaftes auf, um Betriebswärme einer Lichtquelle oder eines Bildsensors von einem distalen Abschnitt abzuleiten. Es sei hierzu angemerkt, dass die Erfindung insbesondere sogenannte "Chip-on-the-Tip"-Endoskope oder -Exoskope betrifft, bei denen die Lichtquelle und/oder der Bildsensor im distalen Abschnitt direkt im Schaft angeordnet ist oder sind.

Dabei sind unterschiedliche Möglichkeiten bekannt, mittels eines jeweiligen Wärmeübertragungselementes ein Ankoppeln der Lichtquelle und/oder des Bildsensors an die Wärmesenke zu ermöglichen.

Allgemein entsteht hierbei jedoch häufig eine Anordnung, in der die Lichtquelle und der Bildsensor in unterschiedlichen Aufnahmeräumen angeordnet sind und damit Abdichtungsprobleme entstehen. Insbesondere beim Sterilisieren solcher medizinischen Bildgebungsvorrichtungen kann es dabei bei einem Versagen einzelner Abdichtungen zu einem Eindringen von beispielsweise Heißdampf kommen, da entsprechende Abdichtungen kompliziert ausgeführt sind und mehrere Dichtebenen umfassen.

Aufgabe der Erfindung ist es, den Stand der Technik zu verbessern.

Gelöst wird die Aufgabe durch eine medizinische Bildgebungsvorrichtung, insbesondere ein Endoskop oder ein Exoskop, mit einem Schaft, einer Lichtquelle zum Beleuchten eines Betrachtungsbereiches und einem Bildsensor zum Aufnehmen des Betrachtungsbereiches, wobei der Schaft einen distalen Abschnitt, einen proximalen Abschnitt, eine vom distalen Abschnitt zum proximalen Abschnitt verlaufende Längsachse, eine an einem distalen Abschnitt angeordnete Stirnfläche und eine entlang der Längsachse verlaufende, einer Umgebung zugewandte Schaftwandung aufweist, die Lichtquelle und der Bildsensor in einem distalen Abschnitt des Grundkörpers aufgenommen sind und eine durch die Lichtquelle und/oder durch den Bildsensor abgegebene Betriebswärme mittels einer Wärmesenke in Richtung eines proximalen Bereichs des Grundkörpers abgeführt wird und der Lichtquelle und dem Bildsensor ein Wärmeübertragungselement zum Leiten der Betriebswärme von der Lichtquelle und/oder vom Bildsensor an die mit dem Wärmeübertragungselement wärmeleitend verbundene Wärmesenke zugeordnet ist, wobei das Wärmeübertragungselement eine erste Kontaktfläche zum Kontaktieren der Lichtquelle und eine zweite Kontaktfläche zum Kontaktieren des Bildsensors aufweist, wobei die Lichtquelle und der Bildsensor mittels eines jeweiligen Kontaktes mit der jeweiligen Kontaktfläche eine jeweilige Betriebswärme an das Wärmeübertragungselement übertragen, sodass die Betriebswärme der Lichtquelle und die Betriebswärme des Bildsensors mittels eines gemeinsamen Wärmeübertragungselementes an die Wärmesenke geleitet werden.

Mit dem Wärmeübertragungselement, welches mit der ersten Kontaktfläche und der zweiten Kontaktfläche sowohl die Lichtquelle als auch den Bildsensor kontaktiert, ist ein Anordnen der Lichtquelle und des Bildsensors in einem gemeinsamen, beispielsweise hermetisch abgeschlossenen, Aufnahmeraum ermöglicht, wobei das gemeinsame Wärmeübertragungselement sodann entsprechende Betriebswärme an die Wärmesenke weiterleiten und abführen kann.

Folgende Begriffe seien in diesem Zusammenhang erläutert:

Eine "medizinische Bildgebungsvorrichtung" kann jede technische und/oder elektrische Einrichtung sein, welche geeignet ist, ein Bild eines Betrachtungsbereiches in einem medizinischen Umfeld aufzunehmen, weiterzuverarbeiten und/oder weiterzuleiten und beispielsweise auf einem Bildschirm anzuzeigen. Beispielsweise ist eine solche medizinische Bildgebungsvorrichtung ein Endoskop oder ein Exoskop. Ein "Endoskop" ist eine zumeist schmale und länglich ausgebildete Bildgebungsvorrichtung, welche geeignet ist, diese Bildgebungsvorrichtung in einen Hohlraum oder durch eine zumeist kleine Öffnung einzuführen und innerhalb des Hohlraums und/oder hinter der kleinen Öffnung angeordnete Bereiche bildtechnisch abzubilden. Endoskope mit einer Anordnung von insbesondere einem Bildsensor im Bereich einer distalen Spitze werden dabei auch als "Chip-on-the-Tip"-Endoskope bezeichnet. Solche Endoskope werden beispielsweise bei medizinischen Eingriffen von außen in den Körper eingeführt und zur Bildgebung innerhalb des Körpers eingesetzt. Ein "Exoskop" bezeichnet eine vergleichbare medizinische Bildgebungsvorrichtung, welche außerhalb eines Körpers eingesetzt wird. In diesem Zusammenhang sei insbesondere darauf hingewiesen, dass ein jeweiliges "Endoskop" fachspezifisch jeweils den dünnen, einführbaren Bereich einer solchen medizinischen Bildgebungsvorrichtung beschreiben kann, jedoch auch für ein Gesamtsystem als Bezeichnung verwendet wird. Dazu kann ein solches Endoskop-System mit weiteren Einrichtungen, beispielsweise einer Kabelführung, weiteren Sensoren und/oder einem Anzeigegerät zum Anzeigen einer Bildinformation auf einen externen Monitor vorliegen. Dabei werden "Endoskop" und "Endoskop-System" häufig unscharf getrennt und teilweise synonym verwendet. Gleiches gilt für Exoskope.

Ein "Schaft" bezeichnet dabei einen, insbesondere häufig metallisch ausgebildeten, länglichen und sehr schlanken Bereich der Bildgebungsvorrichtung, insbesondere eines Endoskopes, welcher beispielsweise einen zum Greifen eines Endoskopes verwendeten Griff mit dem eigentlichen distalen Abschnitt an einer Spitze verbindet. Gleiches gilt analog für andere medizinische Bildgebungsvorrichtungen. Ein solcher Schaft kann dabei beispielsweise als rohrartiger Körper aus einem Edelstahl gebildet sein.

Eine "Lichtquelle" ist beispielsweise eine LED, eine Glühlampe oder eine andere, lichtemittierende Einrichtung. Im Zusammenhang der Erfindung sind dabei spezifisch auch solche Lichtquellen bezeichnet, welche durch eine Anordnung einer LED, mehrerer LEDs oder einer anderen lichterzeugenden Einrichtung im distalen Abschnitt der medizinischen Bildgebungseinrichtung ausgestattet sind. Mittels der Lichtquelle kann Licht an einen entsprechenden Ort im Betrachtungsbereich gelenkt werden, sodass die Lichtquelle insbesondere zum Beleuchten des Betrachtungsbereiches mit Licht oder auch Lichtspektren dient. Es sei hierzu erwähnt, dass die Lichtquelle dabei auch mehrere Teillichtquellen, beispielsweise unterschiedlich gefärbte LEDs oder andere Leuchtmittel aufweisen kann, die beispielsweise für die Beleuchtung des Betrachtungsbereiches mit unterschiedlichen Farben und/oder aus unterschiedlichen Perspektiven eingerichtet sind und auch in einem räumlichen Abstand voneinander und/oder in einem Winkel zueinander angeordnet sein können.

Ein "Betrachtungsbereich" beschreibt den Bereich, das Volumen oder auch einen Flächenabschnitt, welcher oder welches mittels der medizinischen Bildgebungsvorrichtung betrachtet und aus welcher oder welchem ein entsprechendes Bild erzeugt oder abgebildet werden soll. Ein solcher Betrachtungsbereich ist dabei beispielsweise ein Organ, ein Knochen, ein Teilbereich eines menschlichen oder tierischen Körpers oder ein weiterer Bereich von Interesse für eine entsprechende Betrachtung.

Ein "Bildsensor" ist beispielsweise ein elektronischer Chip oder eine andere gleichartige Einrichtung, mittels derer entlang eines optischen Weges ein Bild aus dem Betrachtungsbereich aufgezeichnet und in elektronische Signale umgewandelt werden kann. Beispielsweise ist ein solcher Bildsensor ein CCD-Chip oder ein vergleichbares elektronisches Bauteil. Im Bezug zur Erfindung ist der Bildsensor dabei insbesondere gemeinsam mit der Lichtquelle im distalen Abschnitt angeordnet. Ebenso sei erwähnt, dass der Bildsensor auch eine Anordnung aus mehreren und/oder unterschiedlichen Bildsensoren sein kann, beispielsweise um einen Betrachtungsbereich aus unterschiedlichen Perspektiven und/oder in Bezug zu unterschiedlichen Wellenlängen des Lichts zu betrachten.

Ein "distaler Abschnitt" bezeichnet hierbei einen vom Bediener oder Benutzer des Endoskopes oder Exoskopes oder einer anderen medizinischen Bildgebungsvorrichtung beabstandeten und abgewandten Bereich des Schaftes. Demgegenüber bezeichnet ein "proximaler Abschnitt" den einem Bediener der medizinischen Bildgebungsvorrichtung zugewandten Abschnitt der medizinischen Bildgebungsvorrichtung und/oder des Schaftes. Eine "Längsachse" ist dabei eine Verbindungsachse zwischen distalem Abschnitt und proximalem Abschnitt, wobei diese Längsachse auch als Hauptachse der medizinischen Bildgebungsvorrichtung bezeichnet werden kann und keinesfalls mathematisch exakt definiert sein muss. Die Längsachse bildet hierbei vielmehr den wesentlichen Verlauf der medizinischen Bildgebungseinrichtung, insbesondere entlang des Schaftes, ab.

Eine "Stirnfläche" bezeichnet in diesem Zusammenhang eine am distalen Abschnitt angeordnete und den Schaft und/oder die medizinische Bildgebungsvorrichtung in Richtung des Betrachtungsbereiches abschließende Fläche oder Ebene. Dabei kann die Stirnfläche sowohl orthogonal zur Längsachse als auch in einem Winkel zur Längsachse angeordnet sein. Beispielsweise kann eine Normale der Stirnfläche um 30° zur Längsachse geneigt sein, um eine um 30° in Richtung einer Seite abweichende Blickrichtung zu realisieren. Andere Winkel sind in diesem Zusammenhang möglich. Sofern beispielsweise mehrere Teillichtquellen und/oder mehrere Bildsensoren vorhanden sind, bezeichnet die Stirnfläche dabei beispielsweise ein in einer Haupt-Beleuchtungsrichtung und/oder in eine Haupts-Betrachtungsrichtung ausgerichtete Fläche.

Der Schaft selbst weist eine "Schaftwandung" auf, welche den Schaft physisch in Richtung einer Umgebung begrenzt und beispielsweise im Falle eines rohrförmigen Schaftes eine Zylinderfläche abbildet, wobei der dazugehörige Zylinder eine Mittelachse kongruent zur Längsachse aufweist. In Teilbereichen kann die Schaftwandung dabei auch von einer globalen Geometrie abweichen, beispielsweise Anschlussflächen oder Einschnürungen aufweisen.

"Betriebswärme" bezeichnet die von der Lichtquelle und/oder dem Bildsensor ausgehende Temperaturerhöhung, welche beispielsweise durch eine Verlustleistung der Lichtquelle und/oder des Bildsensors erzeugt und/oder abgegeben wird. Die Betriebswärme wird dabei an eine "Wärmesenke", also an ein Element mit beispielsweise hoher Wärmeleitfähigkeit und/oder hoher Wärmekapazität, abgegeben. Eine solche Wärmesenke kann dabei beispielsweise insbesondere als "Heatpipe" ausgeführt sein, also als thermodynamisch aktives Leitelement zum Fördern von Wärme von einer Wärmequelle in Richtung einer wärmeaufnahmefähigen Struktur der medizinischen Bildgebungsvorrichtung. Dabei wird die Betriebswärme vom distalen Bereich des Schaftes, in dem die Lichtquelle und der Bildsensor angeordnet sind, in Richtung des proximalen Bereichs des Schaftes abgeführt.

Ein "Wärmeübertragungselement" ist ein beispielsweise aus einem wärmeleitfähigen Metall gebildetes technisches Element, an dem die Lichtquelle und/oder der Bildsensor anliegen, um entsprechende Betriebswärme zunächst in das Wärmeübertragungselement und vom Wärmeübertragungselement an die Wärmesenke abzugeben. Dabei weist das Wärmeübertragungselement erfindungsgemäß eine jeweilige erste Kontaktfläche und zweite Kontaktfläche auf, wobei die jeweilige "Kontaktfläche" zum physischen Kontaktieren des Wärmeübertragungselementes mittels der Lichtquelle und mittels des Bildsensors ausgebildet ist. Beispielsweise handelt es sich dabei um eine entsprechend geschaffene metallische Fläche am Wärmeübertragungselement, welche korrespondierend zu einer Seite der Lichtquelle oder zu einer Rückseite des Bildsensors ausgebildet ist, sodass beispielsweise eine möglichst hohe geometrische Kontaktierungsfläche erzielt ist. Der "Kontakt" ist dabei das physische Anliegen der Lichtquelle und des Bildsensors an der jeweiligen Kontaktfläche. Hierzu sei erwähnt, dass bei einer Anordnung mit mehreren Teillichtquellen und/oder mehreren Bildsensoren jeweilige Kontaktflächen oder weitere Kontaktflächen zum physischen Kontaktieren der Teillichtquellen und/oder der Bildsensoren vorhanden sein können. Es ist hierbei einer der Gedanken der Erfindung, dass die jeweilige Lichtquelle oder die jeweiligen Lichtquellen und/oder der Bildsensor oder die jeweiligen Bildsensoren physisch kontaktierend an einem gemeinsamen Wärmeübertragungselement thermisch angekoppelt sind.

Insbesondere können damit die Lichtquelle und der Bildsensor in einem gemeinsamen Aufnahmeraum im distalen Abschnitt angeordnet sein, wobei das Wärmeübertragungselement zumindest teilweise im gemeinsamen Aufnahmeraum angeordnet ist.

Der gemeinsame "Aufnahmeraum" beschreibt dabei einen beispielsweise durch entsprechende Dichtelemente gemeinsam abgedichtetes Volumen, in dem die Lichtquelle und der Bildsensor gemeinsam angeordnet sind, um entsprechende Dichtelemente für jeweils separate Aufnahmeräume einzusparen. Dabei kann beispielsweise das Wärmeübertragungselement auch derart angeordnet sein, dass dieses zumindest teilweise, insbesondere mit der ersten Kontaktfläche und der zweiten Kontaktfläche, in den gemeinsamen Aufnahmeraum hineinreicht und damit zumindest teilweise im gemeinsamen Aufnahmeraum angeordnet ist. Ebenso kann das Wärmeübertragungselement auch vollständig im gemeinsamen Aufnahmeraum angeordnet sein, sodass beispielsweise die Wärmesenke durch eine Begrenzung des Aufnahmeraums geführt ist.

In einer Ausführungsform sind die Lichtquelle und der Bildsensor in einer gemeinsamen Ebene angeordnet, ergänzend oder alternativ kann eine Abschlusslinse oder eine jeweilige Abschlusslinse zum distalen Abschließen des gemeinsamen Aufnahmeraums in der Stirnfläche angeordnet sein.

Damit kann beispielsweise, insbesondere bei einer mit schräger Stirnfläche ausgebildeten medizinischen Bildgebungsvorrichtung, eine besonders kompakte Anordnung erzielt werden.

Eine "gemeinsame Ebene" bezeichnet hierbei eine mathematisch im Wesentlichen kongruent zueinander verlaufende Ebene, in der sowohl die Lichtquelle als auch der Bildsensor mit beispielsweise einer jeweiligen bauteilbedingten Bezugsebene angeordnet sind. Eine "Abschlusslinse" bezeichnet in diesem Zusammenhang beispielsweise eine als Abdeckglas ausgebildete optische Begrenzung, welche gleichsam den distalen Abschnitt der medizinischen Bildgebungsvorrichtung abdichtet.

Um beispielsweise die Herstellung des Wärmeübertragungselementes zu vereinfachen, sind die erste Kontaktfläche und die zweite Kontaktfläche oder auch weitere Kontaktflächen im Wesentlichen parallel zueinander angeordnet, wobei insbesondere die Stirnfläche und die Kontaktfläche aus einer orthogonal zur Längsachse angeordneten Stirnebene geneigt angeordnet sind.

In einer Ausführungsform sind die erste Kontaktfläche und die zweite Kontaktfläche oder auch weitere Kontaktflächen in einem Winkel zueinander angeordnet, wobei insbesondere die erste Kontaktfläche oder die zweite Kontaktfläche parallel zur Stirnfläche angeordnet ist und die jeweilige andere Kontaktfläche im Wesentlichen parallel zur Längsachse angeordnet ist.

Mit dieser Ausgestaltung des Wärmeleitelementes kann eine entsprechend produktionsbedingt einfache Anordnung der Lichtquelle und des Bildsensors an unterschiedlichen Kontaktflächen in unterschiedlichen geometrischen Konstellationen erfolgen.

Insbesondere ist dabei die Lichtquelle oder der Bildsensor an der ersten Kontaktfläche parallel zur Stirnfläche angeordnet und der Bildsensor oder die Lichtquelle parallel zur Längsachse angeordnet, wobei dem jeweiligen parallel zur Längsachse angeordneten Bildsensor oder der jeweiligen parallel zur Längsachse angeordneten Lichtquelle ein Prisma und/oder ein Spiegel zugeordnet ist, wobei mittels des Prismas und/oder mittels des Spiegels ein Umlenken von Licht zwischen der Stirnfläche und dem Bildsensor oder der Lichtquelle ermöglicht ist.

Somit kann eine besonders kompakte Ausgestaltung der medizinischen Bildgebungsvorrichtung, insbesondere mit einem besonders dünnen Schaft, erzielt werden, indem beispielsweise die Lichtquelle oder der Bildsensor "stehend", also parallel zur Stirnfläche angeordnet, und das jeweilig andere Element "liegend" parallel zur Längsachse angeordnet sind.

Ein "Prisma" ist dabei ein optisches Bauelement in Form eines geometrischen Körpers, welches für unterschiedliche optische Effekte eingesetzt werden kann. Beispielsweise kann damit ein Lichtstrahl innerhalb des Prismas umgelenkt werden. Dabei kann beispielsweise auch eine Totalreflexion an einer Innenfläche des Prismas genutzt werden, um eine spiegelartige Umlenkung eines Lichtweges zu erreichen. Demgegenüber ist ein "Spiegel" eine optisch glatte Oberfläche, welche eine Reflexion von Licht ermöglicht und damit ein Umlenken eines Lichtweges, beispielsweise von einem entlang der Längsachse einfallenden Lichtstrahl in Richtung auf einen "liegend" im Schaft angeordneten Bildsensor.

In einer Ausführungsform weist das Wärmeübertragungselement einen der Betriebswärme zugewandten Anschlussbereich und einen der Wärmesenke zugewandten Ableitbereich auf, wobei der Anschlussbereich gegenüber einem Schaftdurchmesser verjüngt ausgebildet ist und der Ableitbereich gegenüber dem Anschlussbereich aufgeweitet ausgebildet ist, sodass ein Anordnen der Lichtquelle oder des Bildsensor in einem vom schlanken Anschlussbereich freigelassenen Einbauquerschnitt des Schaftquerschnittes ermöglicht ist.

Somit ist eine besonders schlanke medizinische Bildgebungsvorrichtung mit einem besonders schlanken Schaft erreichbar.

Der "Anschlussbereich" ist dabei insbesondere dem distalen Abschnitt des Schaftes zugewandt, wobei der "Ableitbereich" dem Ableiten der Betriebswärme in Richtung der Wärmesenke dient und distal angeordnet ist. "Verjüngt" bezeichnet hierbei eine Ausführung mit gegenüber einem anderen Bereich verringertem Querschnitt, womit beispielsweise durch den verjüngt ausgebildeten Anschlussbereich und den demgegenüber aufgeweiteten, also mit größerem Querschnitt ausgestalteten, Ableitbereich eine gestufte Ausführung des Wärmeübertragungselementes erreicht ist. Damit wird mittels des verjüngten Anschlussbereiches ein Einbauquerschnitt für beispielsweise eine Lichtquelle oder den Bildsensor geschaffen.

Um den Wärmeübergang zwischen dem Wärmeübertragungselement und der Wärmesenke zu verbessern, weist das Wärmeübertragungselement am Ableitbereich eine insbesondere sich entlang der Längsachse erstreckende Vertiefung zum Aufnehmen der Wärmesenke auf.

Eine solche "Vertiefung" kann dabei beispielsweise als Bohrung oder andere Materialausnehmung ausgebildet sein, in die beispielsweise die Wärmesenke insbesondere flächig kontaktierend einführbar ist.

In einer Ausführungsform ist die Wärmesenke mittels der Schaftwandung und/oder mittels eines entlang der Längsachse angeordneten Wärmerohres gebildet.

Dabei kann beispielsweise, zumindest zum Teil, die Schaftwandung mit einer der Schaftwandung eigenen Wärmekapazität zum Ableiten von Wärme genutzt werden. Ebenso kann ein "Wärmerohr", also ein auch als "Heatpipe" bekanntes thermodynamisches Element, genutzt werden. Ein solches Wärmerohr nutzt dabei Kapillareffekte und Phasenübergänge eines im Wärmerohr vorhandenen Kühlmediums, um ohne mechanischen Antrieb Wärme zu transportieren. Die Wärmetransportfähigkeit eines solchen Wärmerohres übersteigt dabei insbesondere die Wärmeleitfähigkeit eines beispielsweise Kupferstabes aus Vollmaterial bei weitem.

In einem weiteren Aspekt wird die Aufgabe gelöst durch ein Verfahren zum Steuern und/oder Regeln einer medizinischen Bildgebungsvorrichtungen gemäß einer der vorig bezeichneten Ausführungsformen anhand einer Betriebstemperatur, mit folgenden Schritten:
- Ermitteln einer Betriebstemperatur der Lichtquelle oder einer Betriebstemperatur des Bildsensors mittels eines in der Lichtquelle angeordneten ersten Temperatursensors oder mittels eines im Bildsensor angeordneten zweiten Temperatursensors, sodass eine Betriebstemperatur der Lichtquelle oder eine Betriebstemperatur des Bildsensors ermittelt ist,
- Vergleichen der ermittelten Betriebstemperatur mit einer Vergleichstemperatur, sodass eine Differenz der jeweiligen Betriebstemperatur mit der Vergleichstemperatur vorliegt,
- Steuern oder Regeln einer Lichtleistung der Lichtquelle und/oder einer Bildleistung des Bildsensors anhand der ermittelten Differenztemperatur,
sodass die medizinische Bildgebungsvorrichtung anhand einer Betriebstemperatur gesteuert und/oder geregelt ist.

Insbesondere kann damit erfindungsgemäß die Betriebstemperatur einer medizinischen Bildgebungsvorrichtung, insbesondere eines Endoskopes oder eines Exoskopes, anhand eines beispielsweise in der Lichtquelle oder einer Teillichtquelle oder im Bildsensor oder in einem Bildsensor vorhandenen Temperatursensors geregelt werden, wobei beispielsweise eine gemeinsame Maximaltemperatur dazu genutzt wird, die Lichtleistung der Lichtquelle herabzuregeln, wenn beispielsweise die Betriebstemperatur des Bildsensors eine entsprechende zulässige Vergleichstemperatur übersteigt.

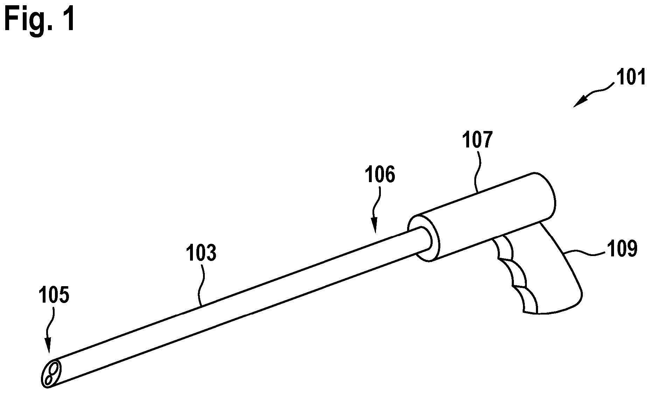

Im Weiteren wird die Erfindung anhand von Ausführungsbeispielen näher erläutert. Es zeigen
- Figur 1: eine schematische Darstellung eines Chip-on-the-Tip-Endoskopes in einer isometrischen Ansicht,

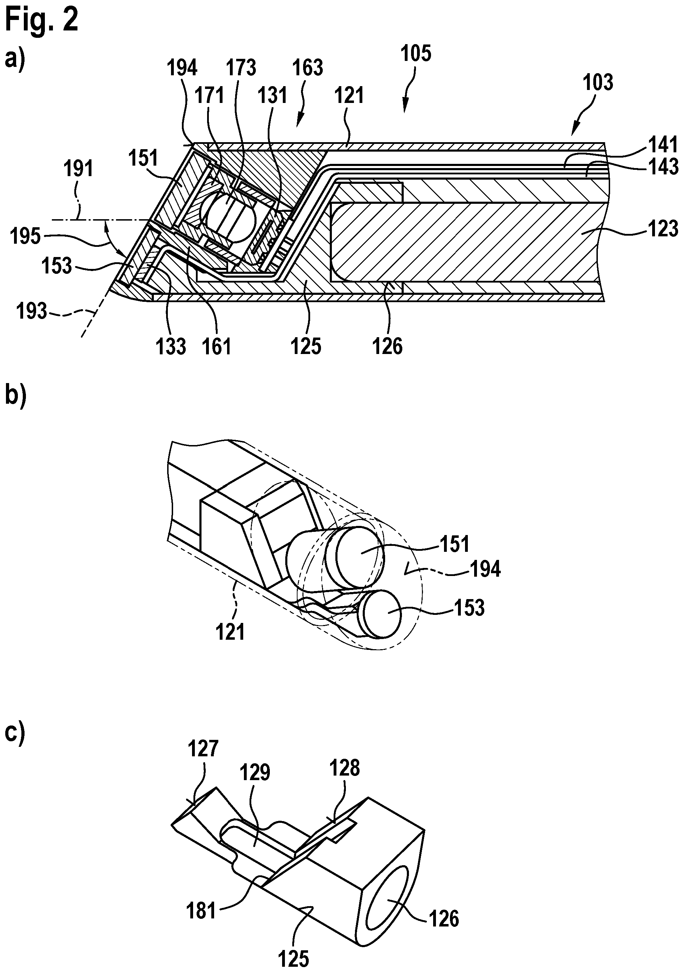

- Figur 2a: eine schematische Darstellung einer Spitze des Endoskopes der Figur 1 in einer geschnittenen Seitenansicht,
- Figur 2b: eine schematische teiltransparente Darstellung der Spitze des Endoskopes der Figur 1 in einer isometrischen Ansicht,
- Figur 2c: eine freigeschnittene Ansicht einer Wärmesenke des Endoskopes der Figur 1 in einer isometrischen Ansicht, sowie

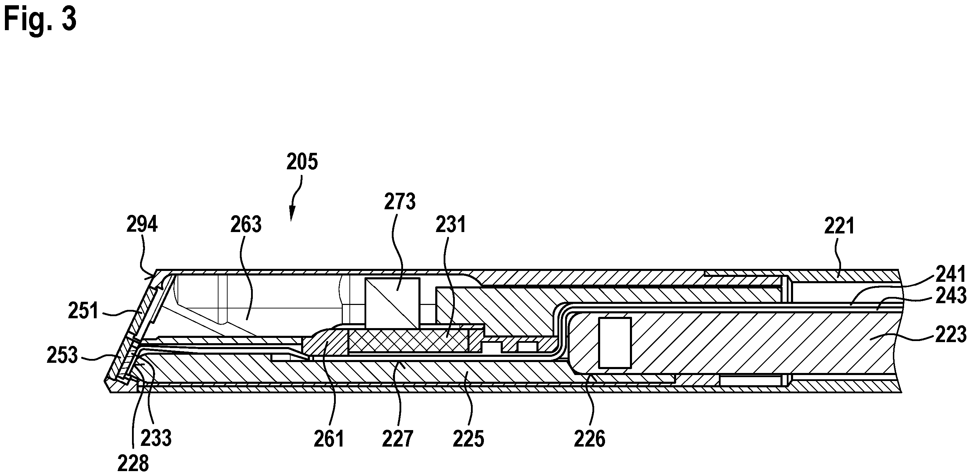

- Figur 3: eine schematische Schnittdarstellung einer alternativen Spitze eines Endoskopes.

Ein Endoskop 101 weist einen Schaft 103 auf, welcher langgestreckt und schlank als Edelstahlrohr ausgeführt ist. Der Schaft 103 weist in einem distalen Bereich eine Spitze 105 auf. In einem proximalen Bereich ist an einem Endbereich 106 ein Griffstück 107 angeordnet, welches einen Griff 109 aufweist. Am Griff 109 kann das Endoskop 101 von einem Bediener geführt werden. Die Spitze 105 kann dann in einen zu betrachtenden Bereich eingeführt werden. Das Griffstück 107 weist weiterhin Bedienelemente sowie Anzeigen auf, welche jedoch vorliegend nicht detailliert beschrieben werden.

Der Schaft 103 weist eine Hülle 121 auf, welche als Edelstahlrohr ausgebildet ist. Die Hülle 121 umschließt in der Spitze 105 die beleuchtenden und bildgebenden Komponenten und sei wie folgt beschrieben:

In der Spitze 105 sind ein Bildsensor 131 sowie eine LED 133 angeordnet. Der Bildsensor 131 erzeugt mittels einer Linse 171 und einer Optik 173 ein Bild aus einem Betrachtungsbereich. Dabei sind der Bildsensor 131, die Linse 171 sowie die Optik 173 durch ein Abdeckglas 151 von einer Umgebung getrennt. Die LED 133 weist ein Abdeckglas 153 auf. Das Abdeckglas 151 sowie das Abdeckglas 153 sind dabei parallel zu einer Stirnebene 193 der Spitze 105 angeordnet, wobei die Stirnebene 193 in einem Winkel 195 gegenüber einer Längsachse 191 entlang des Schaftes 103 angeordnet sind. Damit sind das Abdeckglas 151 und das Abdeckglas 153 in einer Stirnfläche 195 der Spitze 105 angeordnet, wobei die Stirnfläche 194 entlang des Winkels 195 gegenüber dem Schaft 103 geneigt ist. Die Komponenten werden in einem Innenraum 163 in der Spitze 105 von einem Abstandshalter 161 und weiteren, nicht detailliert beschriebenen Bauteilen in Position gehalten.

Rückseitig, in Richtung des distalen Abschnitts, sind die LED 133 sowie der Bildsensor 131 auf einer Wärmesenke 125 angeordnet. Die Wärmesenke 125 weist dazu eine Kontaktfläche 127 für die LED 133 sowie eine Kontaktfläche 128 für den Bildsensor 131 auf. Somit wird die entstehende Betriebswärme der LED 133 und des Bildsensors 131 durch den Kontakt in die Wärmesenke 125 eingeleitet.

Die Wärmesenke 125 ist dabei derart ausgestaltet, dass ein in Richtung der Kontaktfläche 127 führender distaler Abschnitt schlank ausgebildet ist, wohingegen ein die Kontaktfläche 128 stützender Bereich relativ gegenüber dem schlanken Bereich aufgeweitet ausgeführt ist. Distal angeordnet weist die Wärmesenke 125 eine Bohrung 126 auf, in die eine Heatpipe 123 eingeführt ist. Durch die Wärmesenke 125 von der LED 133 und dem Bildsensor 131 abgeführte Wärme wird damit kontaktierend an die Heatpipe 123 abgegeben und in Richtung des distalen Bereichs des Endoskops 101 abgeführt.

Eine Tasche 129 innerhalb der Wärmesenke dient dem Aufnehmen einer Platine 143, welche dem elektrischen Anschluss der LED 133 dient. Weiterhin ist eine Platine 141 dafür vorgesehen, den Bildsensor 131 elektrisch zu kontaktieren. Beide Platinen 141 und 143 sind als flexible Platinen ausgestaltet und führen in Richtung des Griffstückes 107, um die LED 133 sowie den Bildsensor 131 elektrisch zu versorgen und anzusteuern.

Eine alternative Spitze 205, welche beispielsweise als alternative Ausgestaltung des Endoskopes 101 verwendet werden kann, ist in einer Hülle 221 eines Austauschschaftes angeordnet.

Eine Heatpipe 223 dient dem Abführen von Wärme von einer LED 233 und einem Bildsensor 231.

Dazu ist eine Wärmesenke 225 mit einer Anschlussfläche 226 mit der Heatpipe 223 verbunden. Die Wärmesenke 225 ist dabei langgestreckt entlang einer Längsachse der Hülle 221 angeordnet und kontaktiert mit einer Kontaktfläche 228 die LED 233. Dabei ist die Kontaktfläche 228 geneigt parallel zu einer schrägen Stirnfläche 294 angeordnet. In der Stirnfläche 294 ist analog zum vorigen Beispiel mittels eines Abdeckglases 251 ein bildgebender Bereich abgedeckt und mittels eines Abdeckglases 253 die LED 233.

Der Bildsensor 231 ist dabei "liegend", also parallel zu einer Längsachse der Hülle 221, angeordnet. Durch das Abdeckglas 251 einfallende Lichtinformation wird dabei entlang eines Innenraums 263 und durch ein Prisma 273 um 90° auf den Bildsensor 231 umgelenkt.

Wird nun das Endoskop 101, entweder mit der Spitze 105 oder der Spitze 205, in Betrieb genommen, so wird ein Temperatursensor innerhalb des jeweiligen Bildsensors dazu genutzt, eine Gesamttemperatur in der jeweiligen Spitze zu überwachen. Wird nun, beispielsweise durch ein Überschreiten einer Grenztemperatur, ein Überhitzen des Bildsensors 231 festgestellt, so kann die im Bildsensor 231 gemessene Temperaturinformation dazu genutzt werden, beispielsweise auch die jeweilige LED in ihrer Leistung herabzuregeln.

### Bezugszeichenliste

- 101: Endoskop
- 103: Schaft
- 105: Spitze
- 106: Endbereich
- 107: Griffstück
- 109: Griff
- 121: Hülle
- 123: Heatpipe
- 125: Wärmesenke
- 126: Bohrung
- 127: Kontaktfläche
- 128: Kontaktfläche
- 129: Tasche
- 131: Bildsensor
- 133: LED
- 141: Platine
- 143: Platine
- 151: Abdeckglas
- 153: Abdeckglas
- 161: Abstandhalter
- 163: Innenraum
- 171: Linse
- 173: Optik
- 181: Kante
- 191: Längsachse
- 193: Stirnebene
- 194: Stirnfläche
- 195: Winkel
- 205: Spitze
- 221: Hülle
- 223: Heatpipe
- 225: Wärmesenke
- 226: Anschlussfläche
- 227: Kontaktfläche
- 228: Kontaktfläche
- 231: Bildsensor
- 233: LED
- 241: Platine
- 243: Platine
- 251: Abdeckglas
- 253: Abdeckglas
- 261: Abstandhalter
- 263: Innenraum
- 273: Prisma
- 294: Stirnfläche

## Patentansprüche

1. Medizinische Bildgebungsvorrichtung (101), insbesondere Endoskop (101) oder Exoskop, mit einem Schaft (103), einer Lichtquelle (133, 233) zum Beleuchten eines Betrachtungsbereiches und einem Bildsensor (131, 231) zum Aufnehmen des Betrachtungsbereiches, wobei der Schaft (103) einen distalen Abschnitt (105, 205), einen proximalen Abschnitt (106), eine vom distalen Abschnitt (105, 205) zum proximalen Abschnitt (106) verlaufende Längsachse (191), eine am distalen Abschnitt (105, 205) angeordnete Stirnfläche (194, 294) und eine entlang der Längsachse (191) verlaufende, einer Umgebung zugewandte Schaftwandung (121, 221) aufweist, die Lichtquelle (133, 233) und der Bildsensor (131, 231) im distalen Abschnitt (105, 205) des Schaftes (103) aufgenommen sind und eine durch die Lichtquelle (133, 233) und/oder durch den Bildsensor (131, 231) abgegebene Betriebswärme mittels einer Wärmesenke (123, 223) in Richtung eines proximalen Bereiches (106) des Schaftes (103) abgeführt wird und der Lichtquelle (133, 233) und dem Bildsensor (131, 231) ein Wärmeübertragungselement (125, 225) zum Leiten der Betriebswärme von der Lichtquelle (133, 233) und/oder vom Bildsensor (131, 231) an die mit dem Wärmeübertragungselement (125, 225) wärmeleitend verbundene Wärmesenke (123, 223) zugeordnet ist, **dadurch gekennzeichnet, dass** das Wärmeübertragungselement (125, 225) eine erste Kontaktfläche (127, 227) zum Kontaktieren der Lichtquelle (133, 233) und eine zweite Kontaktfläche (128, 228) zum Kontaktieren des Bildsensors (131, 231) aufweist, wobei die Lichtquelle (133, 233) und der Bildsensor (131, 231) mittels eines jeweiligen Kontaktes mit der jeweiligen Kontaktfläche (127, 128, 227, 228) eine jeweilige Betriebswärme an das Wärmeübertragungselement (125, 225) übertragen, sodass die Betriebswärme der Lichtquelle (133, 233) und die Betriebswärme des Bildsensors (131, 231) mittels eines gemeinsamen Wärmeübertragungselementes (125, 225) an die Wärmesenke (123, 223) geleitet werden.

2. Medizinische Bildgebungsvorrichtung gemäß Anspruch 1, **dadurch gekennzeichnet, dass** die Lichtquelle (133, 233) und der Bildsensor (131, 231) in einem gemeinsamen Aufnahmeraum (163, 263) im distalen Abschnitt (105, 205) angeordnet sind, wobei das Wärmeübertragungselement (125, 225) zumindest teilweise im gemeinsamen Aufnahmeraum (163, 263) angeordnet ist.

3. Medizinische Bildgebungsvorrichtung gemäß Anspruch 1 oder 2, **dadurch gekennzeichnet, dass** die Lichtquelle (133, 233) und der Bildsensor (131, 231) in einer gemeinsamen Ebene angeordnet sind und/oder eine Abschlusslinse (151, 153, 251, 253) oder eine jeweilige Abschlusslinse (151, 153, 251, 253) zum distalen Abschließen des gemeinsamen Aufnahmeraumes (163, 263) in der Stirnfläche (194, 294) angeordnet ist oder sind.

4. Medizinische Bildgebungsvorrichtung gemäß einem der vorherigen Ansprüche, **dadurch gekennzeichnet, dass** die erste Kontaktfläche (127, 227) und die zweite Kontaktfläche (128, 228) im Wesentlichen parallel zueinander angeordnet sind, wobei insbesondere die Stirnfläche (194, 294) und die Kontaktflächen (127, 128, 227, 228) aus einer orthogonal zur Längsachse (191) angeordneten Stirnebene (194, 294) geneigt angeordnet sind.

5. Medizinische Bildgebungsvorrichtung gemäß einem der vorherigen Ansprüche, **dadurch gekennzeichnet, dass** die erste Kontaktfläche (127, 227) und die zweite Kontaktfläche (128, 228) in einem Winkel zueinander angeordnet sind, wobei insbesondere die erste Kontaktfläche (127, 227) oder die zweite Kontaktfläche (128, 228) parallel zur Stirnfläche (194, 294) angeordnet ist und die jeweilige andere Kontaktfläche (127, 128, 227, 228) im Wesentlichen parallel zur Längsachse (191) angeordnet ist.

6. Medizinische Bildgebungsvorrichtung gemäß einem der vorherigen Ansprüche, **dadurch gekennzeichnet, dass** die Lichtquelle (133, 233) oder der Bildsensor (131, 231) an der ersten Kontaktfläche (127, 128) parallel zur Stirnfläche (194, 294) angeordnet und der Bildsensor (131, 231) oder die Lichtquelle (133, 233) parallel zur Längsachse (191) angeordnet sind, wobei dem jeweiligen parallel zur Längsachse (191) angeordneten Bildsensor (131, 231) oder der jeweiligen parallel zur Längsachse (191) angeordneten Lichtquelle (133, 233) ein Prisma (273) und/oder ein Spiegel zugeordnet ist, wobei mittels des Prismas (273) und/oder mittels des Spiegels ein Umlenken von Licht zwischen der Stirnfläche (194, 294) und dem Bildsensor (131, 231) oder der Lichtquelle (133, 233) ermöglicht ist.

7. Medizinische Bildgebungsvorrichtung gemäß einem der vorherigen Ansprüche, **dadurch gekennzeichnet, dass** das Wärmeübertragungselement (125, 225) einen der Betriebswärme zugewandten Anschlussbereich (127, 128, 227, 228) und einen der Wärmesenke (123, 223) zugewandten Ableitbereich (126, 226) aufweist, wobei der Anschlussbereich (127, 128, 227, 228) gegenüber einem Schaftdurchmesser verjüngt ausgebildet ist und der Ableitbereich (126, 226) gegenüber dem Anschlussbereich (127, 128, 227, 228) aufgeweitet ausgebildet ist, sodass ein Anordnen der Lichtquelle (133, 233) oder des Bildsensors (131, 231) in einem vom verjüngten Anschlussbereich (127, 128, 227, 228) freigelassenen Einbauquerschnitt (263) des Schaftquerschnittes ermöglicht ist.

8. Medizinische Bildgebungsvorrichtung gemäß einem der vorherigen Ansprüche, **dadurch gekennzeichnet, dass** das Wärmeübertragungselement (125, 225) am Ableitbereich (126, 226) eine insbesondere sich entlang der Längsachse (191) erstreckende Vertiefung (126) zum Aufnehmen der Wärmesenke (123) aufweist.

9. Medizinische Bildgebungsvorrichtung gemäß einem der vorherigen Ansprüche, **dadurch gekennzeichnet, dass** die Wärmesenke (123, 223) mittels der Schaftwandung (121) und/oder mittels eines entlang der Längsachse (91) angeordneten Wärmerohres (123, 223) gebildet ist.

10. Verfahren zum Steuern und/oder Regeln einer medizinischen Bildgebungsvorrichtung (101) gemäß einem der vorherigen Ansprüche 1 bis 9 anhand einer Betriebstemperatur, mit folgenden Schritten:
- Ermitteln einer Betriebstemperatur der Lichtquelle (133, 233) oder einer Betriebstemperatur des Bildsensors (131, 231) mittels eines in der Lichtquelle (133, 233) angeordneten ersten Temperatursensors oder mittels eines im Bildsensor (131, 231) angeordneten zweiten Temperatursensors, sodass eine Betriebstemperatur der Lichtquelle (133, 233) oder eine Betriebstemperatur des Bildsensors (131, 231) ermittelt ist,
- Vergleichen der ermittelten Betriebstemperatur mit einer Vergleichstemperatur, sodass eine Differenz der jeweiligen Betriebstemperatur mit der Vergleichstemperatur vorliegt,
- Steuern oder Regeln einer Lichtleistung der Lichtquelle (133, 233) und/oder einer Bildleistung des Bildsensors (131, 231) anhand der ermittelten Differenztemperatur,
sodass die medizinische Bildgebungsvorrichtung (101) anhand einer Betriebstemperatur gesteuert und/oder geregelt ist.
